# EUROPÄISCHE PATENTSCHRIFT

(11) **EP 0 563 663 B1**
(45) Veröffentlichungstag und Bekanntmachung des Hinweises auf die Patenterteilung: **20.12.1995**
(21) Anmeldenummer: 93104131.3
(22) Anmeldetag: 13.03.1993
(51) Int. Cl.: C07C 309/76, G03F 7/023

(54) **1,2-Naphthochinon-2-diazid-sulfonsäureester, damit hergestelltes strahlungsempfindliches Gemisch und strahlungsempfindliches Aufzeichnungsmaterial**
1,2-naphtoquinone-2-diazidesulfonic acid esters, a radiation-sensitive composition produced therewith and a radiation-sensitive recording material
Esters naphthoquinonique 1,2 de l'acide diazide 2 sulfonique, composition sensible aux radiations obtenue par ces derniers et support d'enregistrement sensible aux radiations

(30) Priorität: 23.03.1992 DE 4209343
(43) Veröffentlichungstag der Anmeldung: 06.10.1993
(73) Patentinhaber: HOECHST AKTIENGESELLSCHAFT, 65926 Frankfurt am Main (DE)
(72) Erfinder: Buhr, Gerhard, Dr., W-6240 Königstein (DE); Zahn, Wolfgang, Dr., W-6228 Eltville 2 (DE); Erdmann, Fritz, Dr., W-6228 Eltville-Martinsthal (DE); Scheler, Siegfried, Dr., W-6200 Wiesbaden-Naurod (DE)

(56) Entgegenhaltungen:
- EP-A- 0 055 814
- EP-A- 0 336 605
- EP-A- 0 351 849
- EP-A- 0 369 219
- PATENT ABSTRACTS OF JAPAN vol. 13, no. 191 (P-867)(3539) 9. Mai 1989

## Beschreibung

Die Erfindung betrifft neue 1,2-Naphthochinon-2-diazid-sulfonsäureester von substituierten Trihydroxybenzophenonen und ein damit hergestelltes strahlungsempfindliches Gemisch mit einem wasserunlöslichen, in wäßrigalkalischen Lösungen dagegen löslichen, zumindest quellbaren, polymeren Bindemittel und mindestens einer strahlungsempfindlichen Verbindung. Sie betrifft weiterhin ein strahlungsempfindliches Aufzeichnungsmaterial mit einem Träger und einer strahlungsempfindlichen Schicht.

Ein wichtiger Schritt bei der Herstellung von elektronischen Bauelementen ist die bildmäßige Bestrahlung und nachfolgende Entwicklung einer strahlungsempfindlichen Schicht, die auf dem zu strukturierenden Material aufgetragen ist. Eine solche Schicht kann positiv oder negativ arbeitend sein.

In einer positiv-arbeitenden Schicht werden allgemein photoaktive Komponenten verwendet, die 1,2-Naphthochinon-2-diazid-Einheiten enthalten. Es ist bereits eine große Anzahl an solchen Komponenten bekannt, vor allem Ester aus aromatischen Polyhydroxyverbindungen und 1,2-Naphthochinon-2-diazid-sulfonsäure. In dem Buch von Jaromir Kosar: "Light-Sensitive Systems", John Wiley + Sons, New York, 1965, Seiten 343 bis 351, ist eine Reihe solcher Ester beschrieben.

Die fortschreitende Miniaturisierung bei der Herstellung von elektronischen Bauelementen erfordert die Erzeugung von immer kleineren Strukturen. Dafür werden strahlungsempfindliche Photoresists mit immer höherem Auflösungsvermögen benötigt. Es ist bekannt, daß durch eine Steigerung des Gehalts an Diazochinon in der strahlungsempfindlichen Schicht die Auflösung verbessert wird (H. Münzel, J. Lux, R. Schulz: A- and B-Parameter dependent Submicron Stepper Performance of Positive Type Photoresist; Microelectronic Engineering 6 (1987), 421-426). P. Trefonas III und B. K. Daniels (New Principle for Image Enhancement in Single Layer Positive Photoresists; Proceedings of SPIE, 771 (1987), 194-210) weisen darauf hin, daß Verbindungen mit mehreren Diazo-naphthochinon-Einheiten stärkere Kontraste und damit eine bessere Auflösung bewirken.

Häufig verwendete und in der Patentliteratur vielfach beschriebene Verbindungen mit mehreren Diazonaphthochinon-Einheiten sind Ester aus 1,2-Naphthochinon-2-diazid-4- und -5-sulfonsäure und Polyhydroxybenzophenon, wobei von den Polyhydroxybenzophenonen das 2,3,4-Trihydroxy-benzophenon bevorzugt ist.

Nachteilig an diesen Estern ist jedoch ihre relativ geringe Löslichkeit in den allgemein verwendeten Lösemitteln, so daß sich ihre Konzentration in den strahlungsempfindlichen Gemischen nicht beliebig erhöhen läßt. Eine weitere Steigerung des Auflösungsvermögens ist daher auf diesem Wege nicht möglich. Zudem sind die mit diesen Estern hergestellten Resistgemische nicht ausreichend lagerstabil.

Diese Nachteile wurden bereits erkannt und in der JP-A 01-017049 beschrieben. Die Lagerstabilität der Resists wurde verbessert durch die Verwendung von Estern aus 1,2-Naphthochinon-2-diazid-5-sulfonsäure und 2,3,4-Trihydroxy-2'-methyl-benzophenon.

Aufgabe der vorliegenden Erfindung ist es daher, Ester aus 1,2-Naphthochinon-2-diazidsulfonsäure und einem 2,3,4-Trihydroxy-benzophenon vorzuschlagen, die eine verbesserte Löslichkeit in den für Photoresists üblichen Lösemitteln aufweisen, in einfacher Weise in chemisch einheitlicher Form herstellbar sind und die lithographischen Eigenschaften der strahlungsempfindlichen Gemische nicht negativ beeinflussen. Außerdem soll durch die Verwendung der neuen Naphthochinondiazide die Lagerstabilität der Resists deutlich verbessert werden. Die Absorptionseigenschaften der Resists sollen durch die neuen Naphthochinondiazide nicht oder nur unwesentlich beeinflußt werden.

Die Aufgabe wird gelöst durch 2,3,4-Trihydroxy-3'-methyl-, -ethyl-, -propyl- oder -isopropyl-benzophenon, das vollständig mit 1,2-Naphthochinon-2-diazid-4-sulfonsäure und/oder 7-Methoxy-1,2-naphthochinon-2-diazid-4-sulfonsäure verestert ist.

Es ist überraschend, daß sich die Aufgabe mit diesen Estern lösen läßt, obwohl in der JP-A 01-017049 festgestellt wird, daß vollständig mit 1,2-Naphthochinon-2-diazid-**5**-sulfonsäure verestertes 2,3,4-Trihydroxy-3'-methyl-benzophenon zu Resistformulierungen mit unzureichender Lagerstabilität führt.

Von den erfindungsgemäßen Verbindungen sind die Ester des 2,3,4-Trihydroxy-3'-methyl-benzophenons bevorzugt.

Hergestellt werden die erfindungsgemäßen Verbindungen nach dem Fachmann bekannten Verfahren. Üblicherweise werden dabei die 3'-alkylsubstituierten 2,3,4-Trihydroxy-benzophenone mit reaktiven Derivaten der 1,2-Naphthochinon-2-diazid-4-sulfonsäure bzw. der 7-Methoxy-1,2-naphthochinon-2-diazid-4-sulfonsäure, insbesondere den Sulfonylchloriden, umgesetzt. Die Umsetzung wird bevorzugt in inerten Lösemitteln, wie Ketonen oder chlorierten Kohlenwasserstoffen, ausgeführt in Gegenwart von anorganischen oder organischen Basen, wie Natriumcarbonat oder tertiären Aminen, z. B. Triethylamin. Die erfindungsgemäßen Naphthochinon-diazid-sulfonsäureester können jedoch auch unter den Bedingungen der Phasen-Transfer-Katalyse, z. B. in einem Zweiphasensystem aus Methylenchlorid und einer wäßrigen Natriumcarbonat- oder Tetraalkylammoniumhydroxid-lösung, mit einem geeignetem Katalysator, wie Tetrabutylammoniumbromid, hergestellt werden.

Gegenstand der vorliegenden Erfindung ist ferner ein strahlungsempfindliches Gemisch mit einem in Wasser unlöslichen, in wäßrig-alkalischen Lösungen dagegen löslichen, zumindest quellbaren polymeren Bindemittel und mindestens einer strahlungsempfindlichen Verbindung, das dadurch gekennzeichnet ist, daß die strahlungsempfindliche Verbindung ein Ester der oben genannten Art ist.

Der Anteil an erfindungsgemäßen Verbindungen beträgt 5 bis 40 Gew.-%, bevorzugt 10 bis 35 Gew.-%, besonders bevorzugt 15 bis 30 Gew.-%, jeweils bezogen auf das Gesamtgewicht der Feststoffe in dem erfindungsgemäßen Gemisch.

Neben den erfindungsgemäßen 1,2-Naphthochinon-2-diazid-4-sulfonsäureestern kann das Gemisch noch andere strahlungsempfindliche Komponenten enthalten. In diesem Fall kann der Anteil der erfindungsgemäßen Ester in dem strahlungsempfindlichen Gemisch in weiten Grenzen schwanken. In einigen Fällen reicht bereits eine geringe Menge an erfindungsgemäßen Estern in einem Gemisch mit anderen strahlungsempfindlichen Komponenten aus, um dem strahlungsempfindlichen Anteil insgesamt eine überraschend höhere Löslichkeit und Lagerstabilität zu verleihen. Im allgemeinen beträgt der Anteil der erfindungsgemäßen Ester 5 bis 99 Gew.-%, vorzugsweise 20 bis 80 Gew.-%, besonders bevorzugt 30 bis 70 Gew.-%, jeweils bezogen auf das Gesamtgewicht aller strahlungsempfindlichen Komponenten.

Als weitere strahlungsempfindliche Komponenten kommen vor allem vollständig oder teilweise mit Naphthochinondiazid-sulfonsäure veresterte Tri- oder Tetrahydroxy-benzophenone in Frage.

Die erfindungsgemäßen strahlungsempfindlichen Gemische enthalten ferner ein polymeres, wasserunlösliches harzartiges Bindemittel, das sich in den für das erfindungsgemäße Gemisch verwendeten Lösemitteln löst und in wäßrigen Alkalien ebenfalls löslich oder zumindest quellbar ist.

Die bei vielen Positiv-Kopiermaterialien als Bindemittel bewährten Novolak-Kondensationsharze haben sich auch in den erfindungsgemäßen Gemischen als besonders brauchbar und vorteilhaft erwiesen. Als eine Ausgangskomponente zu ihrer Herstellung können Phenole, Kresole und Xylenole und allgemein Alkylphenole verwendet werden. Die zweite Komponente sind Aldehyde oder Ketone. Art und Anteil der Novolak-Harze im Gemisch können je nach Anwendungszweck verschieden sein. Im allgemeinen liegt der Novolak-Anteil zwischen 60 und 95 Gew.-%, bevorzugt zwischen 65 und 90 Gew.-%, besonders bevorzugt zwischen 70 und 85 Gew.-%, jeweils bezogen auf das Gesamtgewicht des Feststoffanteils im Gemisch.

Ebenfalls als Bindemittel geeignet sind polymere Verbindungen mit seitenständigen Hydroxyphenylgruppen. Diese enthalten insbesondere Monomereinheiten aus Vinylphenolen und Estern und Amiden aus Acrylsäure und Methacrylsäure mit Polyhydroxy- bzw. Aminohydroxy-aromaten, wie Hydrochinon, Brenzkatechin, Resorcin, Pyrogallol und Hydroxyanilin. Neben den Homopolymeren sind auch Copolymere geeignet. Die Copolymeren können noch weitere Monomereinheiten enthalten, z. B. solche aus Styrol, Methacrylsäureester, Acrylsäureester, Biphenylol-methacrylat oder Biphenylol-acrylat. Auch Mischungen dieser Polymere mit Novolaken sind einsetzbar. Die Art und Anteil der Bindemittel im Gemisch kann je nach Anwendungszweck verschieden sein, der Anteil entspricht jedoch weitgehend dem für die Novolake angegebenen.

Ferner können den erfindungsgemäßen strahlungsempfindlichen Gemischen gegebenenfalls noch weitere Substanzen, wie Farbstoffe, Weichmacher, Netzmittel, Haftvermittler etc., für spezielle Erfordernisse zugesetzt werden.

Zur Beschichtung eines Trägermaterials, z.B. für die Herstellung des erfindungsgemäßen Kopiermaterials, werden die Gemische im allgemeinen in einem Lösemittel gelöst. Die Wahl der Lösemittel ist auf das vorgesehene Beschichtungsverfahren, die Schichtdicke und die Trocknungsbedingungen abzustimmen. Geeignete Lösemittel sind insbesondere Ketone, wie Butanon, N-Methyl-pyrrolidon, Glykolmonoether, wie Ethylenglykol-monomethylether, Propylenglykol-monomethyl- und -monoethylether, Glykoletheracetate, wie Ethylenglykol-ethylether-acetat und Propylenglykol-alkylether-acetate, und Ester, wie Butylacetat. Es können auch Lösemittelgemische verwendet werden. In den Gemischen können auch noch Aromaten, wie Xylol, enthalten sein. Prinzipiell sind alle Lösemittel(gemische) verwendbar, die ein ausreichendes Lösevermögen besitzen und mit den Schichtkomponenten nicht irreversibel reagieren.

Die erfindungsgemäßen Gemische lassen sich als strahlungsempfindliche Komponente in Kopiermaterialien, insbesondere in Photoresists für die Herstellung von Microchips, verwenden. Bevorzugte Schichtträger sind Silicium-Scheiben, die auch oberflächlich oxidiert sein können. Ebenfalls geeignet sind Substrate aus Siliciumnitrid, Polysilicium, Siliciumoxid, Polyimide oder Metalle, wie Aluminium, mit geeigneten Materialien dotiertes Silicium und Scheiben aus Ga/As-Legierungen.

Weiterhin können die erfindungsgemäßen Gemische in der Leiterplattenfertigung verwendet werden. Schließlich lassen sich damit auch strahlungsempfindliche Druckplatten herstellen. Als Schichtträger kommen hier insbesondere Aluminium-Platten, die eine geeignete Vorbehandlung erfahren haben, in Betracht.

Teil der Erfindung ist daher auch ein strahlungsempfindliches Aufzeichnungsmaterial mit einem Schichtträger und einer strahlungsempfindlichen Schicht, das dadurch gekennzeichnet ist, daß die Schicht aus dem oben genannten, erfindungsgemäßen Gemisch besteht.

Die Beschichtung der in der Mikroelektronik-Industrie üblichen Schichtträger erfolgt vorteilhaft durch Aufschleudern. Es können jedoch auch andere Beschichtungstechniken, wie Sprühen, Walzen, Tauchen, Auftragen mittels Breitschlitzdüsen, Rakeln oder Gießer-Antrag, angewendet werden.

Während des Entwicklungsprozesses werden die von der Strahlung getroffenen Bereiche der strahlungsempfindlichen Schicht entfernt, wobei ein positives Abbild der Vorlage zurückbleibt. Entwickelt wird in wäßrig-alkalischen Lösungen. Diese können metallionenfrei sein, aber auch Metallionen, wie Natrium und/oder Kalium, enthalten. Die Entwicklerlösungen können gepuffert sein, z. B. mit Silikat-, Borat- oder Phosphat-Lösungen oder geeigneten Mischungen von Salzlösungen. Sie können vorteilhaft auch geringe Mengen von Tensiden enthalten.

Bei geeigneter Zusammensetzung des strahlungsempfindlichen Gemisches können auch negative Abbilder erzeugt werden, z. B. dann, wenn die durch eine Vorlage bestrahlte Schicht erhitzt, bei Bedarf unter Begasung mit einem Amin, dann ganzflächig bestrahlt und anschließend erst entwickelt wird.

Bevorzugte Anwendung finden die erfindungsgemäßen, strahlungsempfindlichen Gemische in der Herstellung von integrierten Schaltungen oder von diskreten Bauelementen. Sie dienen dabei als Maskierungsmaterial für verschiedene Prozeßschritte, wie Ätzen des Schichtträgers, Implantieren von Ionen in den Schichtträger oder Abscheiden von Materialien auf den Schichtträger.

Wichtige lithographische Beurteilungskriterien sind u. a. die Auflösung eines Resists, d. h. die kleinsten Strukturen, die mit einem Resist noch erzeugt werden können, der Verarbeitungsspielraum, d. h. die Änderung der Strukturdimensionen bei Änderung der Bestrahlungsenergie, und die Strahlungsempfindlichkeit, d. h. die Bestrahlungsenergie, die notwendig ist, um die Strukturen der Maskenvorlage durch den lithographischen Prozeß dimensionsgetreu im Resist wiederzugeben. Für eine reproduzierbare Verarbeitung der Resists ist es wesentlich, daß sich zum einen die lithographischen Kennwerte während der Lagerung des Resists nicht verändern und zum anderen während der Lagerung auch keine Partikel (Kristalle oder Gelteilchen) aus dem Resist ausgeschieden werden, die die Gleichmäßigkeit der Resistschicht stören würden. Derartige Partikel führen zu Fehlstellen in der Resistschicht, was eine Verringerung der Ausbeute bei dem lithographischen Prozeß zur Folge hat. Außerdem bewirken solche Partikel in den Filtern der Pumpen, die den Resist zu der Beschichtungsmaschine transportieren, häufig Verstopfungen, was einen vermehrten Filterwechsel erforderlich macht.

Die erfindungsgemäßen Ester führen zu Gemischen, die zumindest gleich gute lithographische Eigenschaften aufweisen wie Gemische, die Naphthochinondiazid-4-sulfonsäureester des unsubstituierten 2,3,4-Trihydroxy-benzophenons enthalten, jedoch eine wesentlich verbesserte Lagerstabilität besitzen.

Im folgenden werden Beispiele für erfindungsgemäße Gemische genannt, wobei der Erfindungsgedanke jedoch nicht auf diese Beispiele beschränkt sein soll.

### Beispiel 1

### a) 2,3,4-Trihydroxy-3'-methyl-benzophenon

Zu 13,6 g 3-Methyl-benzoesäure in 14,4 ml Bortrifluoridetherat werden portionsweise 12,6 g Pyrogallol gegeben. Das Reaktionsgemisch wird 2,5 h auf 80 °C erwärmt, läßt es abkühlen, fügt während des Abkühlens 50 ml Wasser hinzu und extrahiert dann mit Methylenchlorid. Die organische Phase wird neutralisiert und getrocknet. Dann wird das Lösemittel abgezogen. Zurück bleiben 13,1 g an 2,3,4-Trihydroxy-3'-methyl-benzophenon, das aus Wasser umkristallisiert werden kann. Es fällt in Form von blaßgelben Kristallen mit einem Schmelzpunkt von 108,5 bis 110°C an.
**Verbrennungsanalyse:**

| | % theoret. | % gef. |
|---|---|---|
| C | 68,85 | 68,7 |
| H | 4,95 | 4,9 |

**¹H-NMR (in DMSO-d₆):** 2,40 ppm s, 3 H, -CH₃
6,49 ppm d, 1 H, aromat. Proton in der Trihydroxyphenylgruppe
6,99 ppm d, 1 H, aromat. Proton in der Trihydroxyphenyl-gruppe
7,43 ppm m, 4 H, aromat. Protonen in der Tolylgruppe
**IR-Analyse (KBr-Preßling):** 1632 cm⁻¹ C=O-Bande

### b) Vollständig mit 1,2-Naphthochinon-2-diazid-4-sulfonsäure verestertes 2,3,4-Trihydroxy-3'-methyl-benzophenon

Zu 12,09 g 1,2-Naphthochinon-2-diazid-4-sulfonylchlorid und 3,66 g 2,3,4-Trihydroxy-3'-methyl-benzophenon in 100 ml Acetonitril werden langsam 4,55 g N-Methyl-morpholin getropft. Nach 1,5 Stunden bei Raumtemperatur gießt man das Reaktionsgemisch in 800 ml 0,1 n Salzsäure und saugt den entstandenen Niederschlag ab (8,8 g). Es wird ein gelber Feststoff mit einem Schmelzpunkt von ca. 170°C (unter Zersetzung) erhalten (Verbindung 1).
**Verbrennungsanalyse:**

| | isoliert als Monohydrat | |
|---|---|---|
| | % theoret. | % gef. |
| C | 55,11 | 54,9 |
| H | 2,73 | 2,4 |
| N | 8,76 | 8,8 |
| S | 10,03 | 10,0 |

**UV-Analyse (in Ethylglykolacetat):** λₘₐₓ 378 nm
**IR-Analyse (KBr-Preßling):** 1628 cm⁻¹ C=O-Bande
2145 cm⁻¹ C=N=N-Bande

### Beispiel 2 (Verbindung 2)

Wie unter 1b beschrieben wird 2,3,4-Trihydroxy-3'-methyl-benzophenon mit der dreifachen molaren Menge an 7-Methoxy-1,2-naphthochinon-2-diazid-4-sulfonylchlorid zum entsprechenden vollständig veresterten Produkt umgesetzt.

### Beispiel 3 (Verbindung 3)

Wie unter 1b beschrieben wird 2,3,4-Trihydroxy-3'-methyl-benzophenon mit der dreifachen molaren Menge eines Gemisches aus 0,9 Gt 7-Methoxy-1,2-naphthochinon-2-diazid-4-sulfonylchlorid und 0,1 Gt (jeweils bezogen auf die Gesamtmenge an Naphthochinondiazid) 1,2-Naphthochinon-2-diazid-4-sulfonylchlorid zum entsprechenden Ester umgesetzt.

### Beispiel 4 (Verbindung 4)

Wie unter 1b beschrieben werden 0,5 Gt 2,3,4-Trihydroxy-3'-methyl-benzophenon und 0,5 Gt (jeweils bezogen auf die Gesamtmenge an Trihydroxybenzophenon) 2,3,4-Trihydroxybenzophenon mit der dreifachen molaren Menge eines Gemisches aus 0,9 Gt 7-Methoxy-1,2-naphthochinon-2-diazid-4-sulfonylchlorid und 0,1 Gt (jeweils bezogen auf die Gesamtmenge an Naphthochinondiazid) 1,2-Naphthochinon-2-diazid-4-sulfonylchlorid zum entsprechenden vollständig veresterten Produkt umgesetzt.

### Vergleichsbeispiel 1 (Verbindung 5)

Wie unter 1b beschrieben wird 2,3,4-Trihydroxybenzophenon mit der dreifachen molaren Menge an 1,2-Naphthochinon-2-diazid-4-sulfonylchlorid zum entsprechenden Triester umgesetzt.

### Vergleichsbeispiel 2 (Verbindung 6)

Wie unter 1b beschrieben wird 2,3,4-Trihydroxybenzophenon mit der dreifachen molaren Menge an 7-Methoxy-1,2-naphthochinon-2-diazid-4-sulfonylchlorid zum entsprechenden Triester umgesetzt.

### Vergleichsbeispiel 3 (Verbindung 7)

Wie unter 1b beschrieben wird 2,3,4-Trihydroxybenzophenon mit der dreifachen molaren Menge eines Gemisches aus 0,9 Gt 7-Methoxy-1,2-naphthochinon-2-diazid-4-sulfonylchlorid und 0,1 Gt (jeweils bezogen auf die Gesamtmenge an Naphthochinondiazid) 1,2-Naphthochinon-2-diazid-4-sulfonylchlorid zum entsprechenden Triester umgesetzt.

### Vergleichsbeispiel 4 (Verbindung 8)

Wie unter 1b beschrieben wird 2,3,4-Trihydroxy-3'-methyl-benzophenon mit der dreifachen molaren Menge an 1,2-Naphthochinon-2-diazid-5-sulfonylchlorid zum entsprechenden Triester umgesetzt.

### Anwendungsbeispiel 1 (Löslichkeit)

Zum Vergleich der Löslichkeiten der Verbindung 1 und der Verbindung 5 wurden beide Verbindungen in kristalliner Form hergestellt und daraus dann gesättigte Lösungen in Propylenglykolmethyletheracetat bereitet. Der Gehalt der Lösungen wird UV-spektroskopisch bestimmt durch Messung der Extinktion bei λₘₐₓ und Vergleich mit einer Eichgeraden, die aus Lösungen bekannter Konzentration erhalten wurde. Die Löslichkeit der Verbindung 1 beträgt 1,7 Gew.-% und die der Verbindung 5 nur 0,4 Gew.-%, was eine Steigerung der Löslichkeit um den Faktor 4 bedeutet.

### Anwendungsbeispiel 2 (Löslichkeit)

Zur Prüfung der Löslichkeiten der Verbindungen 2, 3, 4, 6, 7 und 8 werden gesättigte Lösungen der Naphthochinondiazide in Propylenglykolmethylether-acetat (PGMEA), das 25 Gew.-% an Novolak enthält, hergestellt. Die so erhaltenen Lösungen zeigen sehr gut die realen Verhältnisse in einem Photoresist. Durch Messung der UV-Absorption bei λₘₐₓ und Vergleich gegen eine Eichgerade wird die Konzentration der gesättigten Lösung bestimmt. Die Ergebnisse sind in Tabelle 1 wiedergegeben.

**Tabelle 1**

| Löslichkeiten der Naphthochinondiazide | |
|---|---|
| Verbindung | Löslichkeit |
| 2 | > 26,1 Gew.-% |
| 3 | > 27,9 Gew.-% |
| 4 | > 39,3 Gew.-% |
| 6 | 6,5 Gew.-% |
| 7 | 8,5 Gew.-% |
| 8 | 3,3 Gew.-% |

### Anwendungsbeispiel 3 (Lagerstabilität und lithographische Eigenschaften)

Zur Bestimmung der Lagerstabilität und der lithographischen Eigenschaften wird aus den erfindungsgemäßen Verbindungen und den Vergleichsverbindungen ein Photoresist bereitet mit der folgenden Formulierung:
5,6 Gt Naphthochinondiazid,
18,7 Gt Novolak (bestehend aus m- und p-Kresol und Xylenolen) mit einem mittleren Molekulargewicht M_{w} von 4200 (gegen einen Polystyrol-Standard),
36,5 Gt Propylenglykol-methylether-acetat.

Als Maß für die Lagerstabilität wird die Zeitdauer bis zur beginnenden Kristallisation der Naphthochinondiazide aus der Resistlösung herangezogen, wobei die Lagerung der Resists bei Raumtemperatur erfolgt. Die Ergebnisse sind in Tabelle 2 aufgezeigt.

Als lithographische Eigenschaften werden in Tabelle 2 die Strahlungsempfindlichkeit (definiert als notwendige Bestrahlungsenergie zur Übertragung der Maskenstrukturen auf den Resist) in relativen Einheiten, der Dunkelabtrag (das ist der Resistabtrag während der Entwicklung in den nicht bestrahlten Bereichen), und der Kontrastwert (als Maß für das Auflösungsvermögen der Resists) angegeben.

In Tabelle 2 sind ebenfalls die Werte der UV-Absorption der Resists bei λₘₐₓ, normiert über die Schichtdicke, aufgeführt.

**Tabelle 2**

| Resist-Eigenschaften | | | | | |
|---|---|---|---|---|---|
| Verbindung Nr. | UV-Abs. λₘₐₓ | Relative Strahlungsempfindlichkeit | Dunkelabtrag nm | Kontrastwert | Lagerstabilität (Tage) |
| 1 | 0,599 | 1,03 | 14 | 1,5 | 17 |
| 2 | 0,409 | 1,05 | 8 | 2,5 | >49 |
| 3 | 0,411 | 1,02 | 7 | 2,4 | >49 |
| 4 | 0,410 | 0,95 | 12 | 2,5 | >49 |
| 5 | 0,616 | 1,00 | 8 | 1,5 | 5 |
| 6 | 0,421 | 0,94 | 10 | 2,1 | 14 |
| 7 | 0,421 | 0,90 | 12 | 2,3 | 16 |

Tabelle 2 zeigt deutlich, daß sich UV-Absorption, Strahlungsempfindlichkeit, Dunkelabtrag und Kontrastwert der erfindungsgemäßen Naphthochinondiazide von den Vergleichsverbindungen praktisch nicht unterscheiden, während die Lagerstabilität der erfindungsgemäßen Naphthochinondiazide erheblich verbessert ist.

## Patentansprüche

1. 2,3,4-Trihydroxy-3'-methyl-, -ethyl-, -propyl- oder -isopropyl-benzophenon, das vollständig mit 1,2-Naphthochinon-2-diazid-4-sulfonsäure und/oder 7-Methoxy-1,2-naphthochinon-2-diazid-4-sulfonsäure verestert ist.

2. 2,3,4-Trihydroxy-3'-methyl-benzophenon, das vollständig mit 1,2-Naphthochinon-2-diazid-4-sulfonsäure und/oder 7-Methoxy-1,2-naphthochinon-2-diazid-4-sulfonsäure verestert ist.

3. Strahlungsempfindliches Gemisch, das als wesentliche Bestandteile ein polymeres, wasserunlösliches, in organischen Lösemitteln und wäßrig-alkalischen Lösungen dagegen lösliches, zumindest quellbares, Bindemittel und mindestens eine strahlungsempfindliche Verbindung enthält, dadurch gekennzeichnet, daß die strahlungsempfindliche Verbindung eine Verbindung gemäß Anspruch 1 ist.

4. Strahlungsempfindliches Gemisch nach Anspruch 3, dadurch gekennzeichnet, daß der Anteil an strahlungsempfindlichen Verbindungen gemäß Anspruch 1 5 bis 40 Gew.-%, bevorzugt 10 bis 35 Gew.-%, besonders bevorzugt 15 bis 30 Gew.-% beträgt, jeweils bezogen auf das Gesamtgewicht der Feststoffe in dem Gemisch.

5. Strahlungsempfindliches Gemisch nach Anspruch 3, dadurch gekennzeichnet, daß es noch andere strahlungsempfindliche Verbindungen enthält und der Anteil der Verbindungen gemaß Anspruch 1 an dem Gesamtgewicht der strahlungsempfindlichen Verbindungen 5 bis 99 Gew.-%, bevorzugt 20 bis 80 Gew.-%, besonders bevorzugt 30 bis 70 Gew.-% beträgt.

6. Strahlungsempfindliches Gemisch nach Anspruch 3, dadurch gekennzeichnet, daß das polymere Bindemittel ein Novolak ist und dieses einen Anteil von 60 bis 95 Gew.-%, bevorzugt 65 bis 90 Gew.-%, besonders bevorzugt 70 bis 85 Gew.-% hat, jeweils bezogen auf das Gesamtgewicht des Feststoffanteils im Gemisch.

7. Strahlungsempfindliches Gemisch gemäß einem oder mehreren der Ansprüche 3 bis 5, dadurch gekennzeichnet, daß das Bindemittel eine polymere Verbindung mit seitenständigen Hydroxyphenylgruppen ist.

8. Strahlungsempfindliches Aufzeichnungsmaterial mit einem Schichtträger und einer strahlungsempfindlichen Schicht, dadurch gekennzeichnet, daß die Schicht aus einem strahlungsempfindlichen Gemisch gemäß einem oder mehreren der Ansprüche 3 bis 7 besteht.

## Claims

1. A 2,3,4-trihydroxy-3'-methyl-, -ethyl-, -propyl- or -isopropylbenzophenone which is completely esterified with (1,2-naphthoquinone 2-diazide)-4-sulfonic acid and/or (7-methoxy-1,2-naphthoquinone 2-diazide)-4-sulfonic acid.

2. A 2,3,4-trihydroxy-3'-methylbenzophenone which is completely esterified with (1,2-naphthoquinone 2-diazide)-4-sulfonic acid and/or (7-methoxy-1,2-naphthoquinone 2-diazide)-4-sulfonic acid.

3. A radiation-sensitive mixture which contains, as essential constituents, a polymeric, water-insoluble binder which is, however, soluble, or at least swellable, in organic solvents and aqueous alkaline solutions and at least one radiation-sensitive compound, wherein the radiation-sensitive compound is a compound as claimed in claim 1.

4. A radiation-sensitive mixture as claimed in claim 3, wherein the proportion of radiation-sensitive compounds as claimed in claim 1 is 5 to 40% by weight, preferably 10 to 35% by weight, particularly preferably 15 to 30% by weight, based in all cases on the total weight of the solids in the mixture.

5. A radiation-sensitive mixture as claimed in claim 3, which contains yet other radiation-sensitive compounds and the proportion of the compounds as claimed in claim 1 in the total weight of the radiation-sensitive compounds is 5 to 99% by weight, preferably 20 to 80% by weight, particularly preferably 30 to 70% by weight.

6. A radiation-sensitive mixture as claimed in claim 3, wherein the polymeric binder is a novolak and the proportion of the latter is 60 to 95% by weight, preferably 65 to 90% by weight, particularly preferably 70 to 85% by weight, based in all cases on the total weight of the solid component in the mixture.

7. A radiation-sensitive mixture as claimed in one or more of claims 3 to 5, wherein the binder is a polymeric compound having lateral hydroxyphenyl groups.

8. A radiation-sensitive recording material comprising a layer substrate and a radiation-sensitive layer, wherein the layer is composed of a radiation-sensitive mixture as claimed in one or more of claims 3 to 7.

## Revendications

1. 2,3,4-trihydroxy-3'-méthyl-, -éthyl-, -propyl- ou -isopropyl-benzophénone, qui est estérifiée totalement par de l'acide 1,2-naphtoquinone-2-diazide-4-sulfonique et/ou de l'acide 7-méthoxy-1,2-naphtoquinone-2-diazide-4-sulfonique.

2. 2,3,4-trihydroxy-3'-méthyl-benzophénone, qui est totalement estérifiée avec de l'acide 1,2-naphtoquinone-2-diazide-4-sulfonique et/ou de l'acide 7-méthoxy-1,2-naphtoquinone-2-diazide-4-sulfonique.

3. Composition sensible aux radiations, qui contient en tant qu'ingrédients essentiels un liant polymère, insoluble dans l'eau, mais par contre soluble ou au moins susceptible de gonfler dans les solvants organiques et les solvants aqueux alcalins, et au moins un composé sensible aux radiations, caractérisée en ce que, le composé sensible aux radiations est un composé selon la revendication 1.

4. Composition sensible aux radiations, selon la revendication 3, caractérisée en ce que la teneur en composé sensible aux radiations selon la revendication 1, est de 5 à 40 % en poids, de préférence 10 à 35 % en poids, plus particulièrement 15 à 30 % en poids, par rapport au poids total de solides dans la composition.

5. Composition sensible aux radiations, selon la revendication 3, caractérisée en ce qu'elle comprend en outre d'autres composés sensibles aux radiations et en ce que la proportion des composés selon la revendication 1 dans le poids total de composés sensibles aux radiations, est de 5 à 99 % en poids, de préférence 20 à 80 % en poids, plus particulièrement 30 à 70 % en poids.

6. Composition sensible aux radiations, selon la revendication 3, caractérisée en ce que le liant polymère est une novolaque et est présent selon un teneur de 60 à 95 % en poids, de préférence de 65 à 90 % en poids, plus particulièrement de 70 à 85 % en poids, par rapport au poids total de solides dans la composition.

7. Composition sensible aux radiations, selon l'une ou plusieurs des revendications 3 à 5, caractérisée en ce que le liant est un composé polymère à groupes hydroxyphényle latéraux.

8. Matériau d'enregistrement sensible aux radiations comportant un support de couches et une couche sensible aux radiations, caractérisé en ce que ladite couche est constituée d'une composition sensible aux radiations selon l'une ou plusieurs des revendications 3 à 7.
